# EUROPEAN PATENT SPECIFICATION

(11) **EP 0 452 421 B1**
(45) Date of publication and mention of the grant of the patent: **08.10.1997**
(21) Application number: 90903510.7
(22) Date of filing: 03.01.1990
(51) Int. Cl.: G01N 33/36, G01N 3/08

(54) **FIBER TESTING METHOD**
FASERTESTVERFAHREN
PROCEDE D'ESSAI DE FIBRE

(30) Priority: 04.01.1989 US 293258
(43) Date of publication of application: 23.10.1991
(73) Proprietor: ZELLWEGER USTER, INC., Knoxville, TN 37950-1270 (US)
(72) Inventor: SHOFNER, Frederick, M., Knoxville, TN 37922 (US); CHU, Youe-T, Knoxville, TN 37922-5560 (US); SHOFNER, Christopher, K., Knoxville, TN 37922 (US); TOWNES, Mark, G., Knoxville, TN 37922 (US)
(74) Representative: Ellenberger, Maurice
(86) International application number: US9000077
(87) International publication number: WO9008306

(56) References cited:
- EP-A- 0 241 894
- AU-B- 0 226 070
- BE-A- 558 530
- DE-A- 3 308 001
- DE-B- 1 276 934
- GB-A- 2 031 960
- GB-A- 2 084 331
- JP-A- 5 611 338
- JP-A-58 211 624
- US-A- 2 537 170
- US-A- 3 049 916
- US-A- 3 069 964
- US-A- 3 290 932
- US-A- 4 031 746
- US-A- 4 173 787
- US-A- 4 511 253
- US-A- 4 562 743
- US-A- 4 634 280
- US-A- 4 891 974
- US-A- 4 895 028
- RESEARCH DISCLOSURE, no. 15782, May 1977, page 83, Havant, GB; "Yarn sampling system"
- Proceedings of the IEEE, Vol. 63, No. 10, October 1975, J.L. DORRITY et al., "A Minicomputer-Controlled all-digital tensile test system", pages 1451-1459.
- RESEARCH DISCLOSURE, No. 13710 September 1975, "Pick-up detector", page 13. See Figure.
- Materials Research and Standards, Vol. 10, No. 6, June 1970; J. SKELTON, "Tensile Behavior of Fibrous Materials at High Rates of Strain and Subambient Temperatures", pages 20-25. See Figures 3 & 4.

## Description

The invention relates to a method for producing multiple fibre data as to fibres taken from a sample composed of fibres, wherein a first test is performed to determine first and second characteristics of at least one fibre taken from the sample and a first representation of the first and second characteristic is produced of said first test, at least a second test is performed to determine first and second characteristics of at least one additional fibre taken from the sample and a second representation of the first and second characteristic is produced of said second test.

For proper utilization of natural or man-made fibers it is essential to have precise, accurate, and basic tensile characteristics of the fibers. To illustrate, in the making of yam from cotton or polyester staple, in the researching genetic influences upon characteristics of cotton or wool, or in the production of man-made fibers from carbon or glass, data relating to the tensile characteristics of the fibers commonly need to be compiled and studied. As used herein, the term "tensile" will be understood to include force-elongation characteristics and cross-sectional characteristics. For example, the traditional material characteristic of tensile stress is simply the ratio of breaking force divided by cross-sectional area; common dimensions for tensile stress are newtons/m^{2.} As a second example, percentage elongation is commonly taken as the elongation for which Hooke's Law holds (when incremental force is directly proportional to incremental elongation) divided by the untensioned length (gauge) of the material times 100%. (Note that this definition of elongation exchanges elongation components when the force is small, i.e. "crisp" or "stack", or when force is not linear with incremental elongation.)

It is desirable that such tensile data be generated quickly and in sufficient quantities to permit the statistical analysis of the fibres.

Known instruments used for obtaining tensile data on a bundle of fibers are not capable of providing data on individual fibers. In the textile industry, it is traditional to test bundles of fibers as opposed to single fibers. In part, the rationale behind this procedure is that fibers are normally used in bundles such as cotton yam (thread). However, these traditional test procedures do not provide much, if any, information about the single fiber. For example, a break test of a fiber bundle will not provide much information about the amount of load each fiber bears during the test or the manner in which each fiber elongates and breaks during the test. Even if the sensitivity of the force transducer is made very high, the data obtained from a fiber bundle test is masked to some extent by damaged oscillatory response of the force transducer or ringing. That is, as individual fibers in a bundle break, a ringing oscillation is inherently set up in th test device that may distort data. Since the fibers of a bundle break at varying elongations, this ringing effect is occurring throughout the majority of the test. Ringing may not significantly affect the data as to the entire bundle, but it is usually sufficient to frustrate any attempt to derive precise data as to individual fiber characteristics or contributions.

Existing instruments for testing a single fiber, such as the Instron force-elongation tester, are very slow to use due to procedures normally involved in preparing the fiber for testing. In the textile industry, individual fiber testing is seldom done because the statistical quality of data thus obtained is not viewed as useful as bundle test data.

GB-A-2 084 311 discloses a measuring instrument for studying the tensile characteristics of hair or other fibres and for carrying out measurements on a series of samples automatically. A statistic evaluation of fibres is thereby performed rapidly, but the values obtained are of the type of mean values related to single fibres. Combining or superposing of characteristics in order to obtain significant values related to a group of combined fibres is not possible with the known instrument. Characteristics of such a group of combined fibres, which may be totally different from the characteristics of a single fibre cannot be obtained.

It is therefore an object of the present invention to provide multiple fibre data belonging to a group of combined fibres.

The present invention resides in a method for producing multiple fiber data which are derived from two-coordinate graphical representations (or arrays) of elongation and force characteristics of individual fibers in the bundle. Within the graphical representations, the magnitude of fiber elongation is plotted along one coordinate, and the magnitude of force exerted upon the fiber is plotted along the other coordinate. For a preselected elongation magnitude, the force values of the graphical representations are added together to obtain an additive force value, and the additive force value is plotted on a secondary two-coordinate graph wherein one coordinate of the secondary graph denotes elongation magnitudes and the other coordinate of the secondary graph denotes a range of additive force values. Force values are added together for a number of other preselected elongation magnitudes identified on the graphical representations and the resultant additive values are plotted on the secondary graph to obtain a secondary graphical representation of force-elongation characteristics for all of the fibers tested. This secondary graphical representation constitutes fiber data.

Multiple fiber data obtained by the present invention is not identical to data obtained from any known bundle test, but there is correlation between multiple fiber data and both twisted fiber bundle test and parallel (brushed) fiber tests. Generally, multiple fiber data predict an upper limit for twisted bundle tests and a lower limit for parallel bundle tests. In some instances, mutiple fiber data may be more useful than bundle tests. For example, if crimp is known to significantly affect performance of fibers in a certain application, then the parallel brushed bundle test would not be appropriate because crimp is uncontrollably removed (brushed out) from the fiber before the test. On the other hand, multiple fiber data normally (but not always) include fiber crimp effects and would be more useful in predicting performance or selecting fibers from this certain applications.

In the above discussion, multiple fiber data was obtained by "adding" force values from a plurality of "graphical representations". It will be understood that "graphical representations" will normally take the form of numbers in a computer, such as a numerical array, and the term graphical representation has been chosen because it best communicates visually the invention. It does not imply that the actual production of a physical graph is necessary to the invention.

Likewise, the term "added" should be understood in a broad sense. While mathematically precise adding is preferred, the graphical representations could be combined or mixed electronically in ways that might not constitute adding in a strict mathematical sense.

The present invention may best be understood by reference to illustrative embodiments which are shown in the accompanying drawings.

Figures 1-3 are graphical representations of test results carried out on individual fiber samples.

Figure 4 is a graphical representation obtained by superimposing information bome by the graphs of Figures 1-3 onto a single graph.

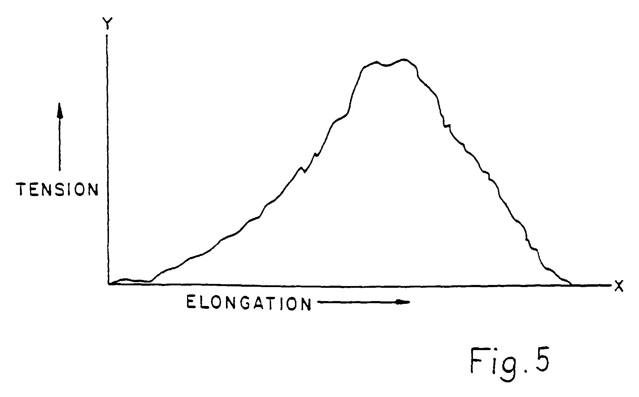

Figure 5 is a graphical representation obtained by superimposing information bome by a large number of graphs like those of Figures 1-4 onto a single graph.

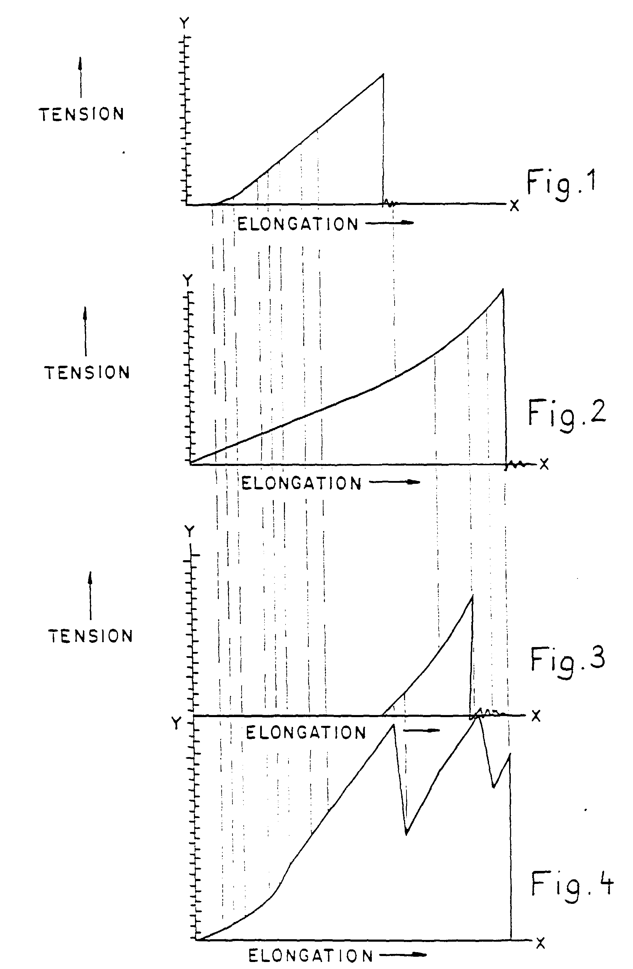

With reference to Figures 1-3, there are shown two-coordinate graphical representations of elongation and tensile data collected when subjecting three fibers to a tensile test. More specifically, Figure 1 is a graphical representation of the test results gathered when tensioning a first fiber until it breaks; Figure 2 is a graphical representation of the test results gathered when tensioning a second fiber until it breaks; and Figure 3 is a graphical representation of the test results gathered when tensioning a third fiber until it breaks. In each graph of Figures 1-3, the magnitude of the fiber elongation is plotted along the abscissa, and the corresponding magnitude of the tensile forces is plotted along the ordinate.

The force-elongation readings are stored in orderd arrays corresponding to ordered pairs (x, y) on each of Figures 1-3. The locus of all such ordered pairs is simply the force-elongation graph. Since equal x-values correspond to identical values of elongation, and since elongation is directly proportional to time, the same x-value corresponds to alignment in time; thus the graphs of Figures 1-3 can be called "time-aligned arrays".

That is, for emphasis, time-aligned force-elongation arrays can be manipulated to model the total force for a multiplicity of fibers. This will now be explained using the graphical representations of Figures 1-3.

In order to predict the tensile characteristics of a bundle of fibers comprised of the three fibers whose test results are graphically illustrated in Figures 1-3, the graphical representations of Figures 1-3 may be superposed on one another to form the graph of Figure 4. To superpose the graphs of Figures 1-3, the tensile stress values of the graphical representations for a preselected elongation magnitude are added together to obtain one additive stress value, and the additive stress value is plotted on a secondary graph (i.e., the graph of Figure 4) wherein a range of stress values is represented along the ordinate of the secondary graph and a range of elongation magnitudes is plotted along the abscissa of the secondary graph. The tensile stress values identified on Figures 1-3 for a number of other preselected elongation magnitudes are also added together and the locus of resulting additive values plotted on the secondary graph is the resulting graphical representation predicted tensile characteristics of the fiber bundle over a relatively broad range of elongation magnitudes.

The information shown in the secondary graph, Figure 4, may be termed multiple fiber data and as explained above it correlates to some extent with fiber bundle test, but it is not duplicative of any known bundle test. While the technique of deriving the secondary graph of Figure 4 has been described with reference to three fibers, in actual practice, many fibers (on the order of 100's) will be tested to provide accurate data as to a particular sample of fibers.

Also, it will be understood that the data shown in Figures 1-3 has been chosen to best illustrate the invention and it does not represent any particular fiber sample. It should be noted that the fiber of Figure 3 elongated for a significant period of time without any tension being produced in the fiber (high crimp), but the fiber of Figure 2 experienced a tension force immediately upon elongation (low crimp). Usually, elongation of a fiber without placing tension forces on the fiber indicates that the fiber was crimped and the initial elongation is removing crimp. These graphs indicate that the Figure 3 fiber had a very large amount of crimp, while the Figure 2 fiber had zero crimp. The difference in the amount of crimp helps illustrate the invention.

The above described method of obtaining multiple fiber data is not limited to tension and elongation data. For example, in an alternate embodiment, cross section data, such as area, diameter or thickness are substituted for elongation and alignment of the data is accomplished according to either time or the cross-sectional data. Also, the multiple fiber data is not limited to two dimensions. For example, one may also produce three coordinate arrays (or three dimensional graphs) using, for example, elongation, tension, and a cross sectional characteristic.

## Claims

1. Method for producing multiple fibre data as to fibres taken from a sample composed of fibres, wherein
a first test is performed to determine first and second characteristics (x, y) of at least one fibre taken from the sample (22) and a first representation of the first and second characteristic is produced of said first test,
at least a second test is performed to determine first and second characteristics (x, y) of at least one additional fibre (22) taken from the sample and a second representation of the first and second characteristic is produced of said second test, characterized in that,
the first and second representations of the first and second characteristics are combined to produce a composite representation of the first and second characteristics for all fibres that were tested, wherein one of said first and second characteristics from said at least second test is superposed to said same characteristic from said first test, said composite representation constituting multiple fibre data.

2. Method according to claim 1, characterised in that said first and second characteristics are constitued by the tension force and the elongation length.

3. Method according to claim 2, characterised in that said first and second representations are constituted by values representing said tension forces and said elongation length.

4. Method according to claim 1 and 2, characterised in that combining the characteristics is performed by adding the tension forces obtained by said first and said at least second test for each one of a plurality of tension lengths.

5. Method according to claim 1, characterised in that said step of combining comprises:
aligning the first and second representations according to the first characteristic so that, for identical characteristics in the first and second representations, the second characteristics will be aligned, and adding the aligned second characteristics of the first and second representations to form the second characteristics in the composite representation.

6. Method according to claim 1, characterised in that said first characteristic is tension on the fibre and said second characteristic is elongation or a cross-sectional characteristic of the fibre.

7. Method according to claim 1, characterised in that a third characteristic of the fibres is determined and in that said first, second and third characteristic is combined to produce the composite representation.

8. Method according to claim 1, characterised in that said first and second characteristics are stored before combining.

9. Method according to claim 1, characterised in that said composite representation of data taken from single fibres used for predicting the characteristics of a bundle of fibres containing a plurality of fibres.

## Patentansprüche

1. Verfahren zum Erzeugen verschiedener Angaben über Fasem, die einer Probe entnommen sind, die aus Fasem besteht, wobei eine erste Prüfung durchgeführt wird, um erste und zweite Eigenschaften (x, y) wenigstens einer Faser aus der Probe zu bestimmen und eine erste Darstellung der ersten und zweiten Eigenschaft aus der ersten Prüfung erfolgt, mindestens eine zweite Prüfung durchgeführt wird, um erste und zweite Eigenschaften (x, y) mindestens einer weiteren Faser (22) aus der Probe zu bestimmen und eine zweite Darstellung der ersten und zweiten Eigenschaft aus der zweiten Prüfung erfolgt, dadurch gekennzeichnet, dass die erste und die zweite Darstellung der ersten und zweiten Eigenschaften kombiniert werden, um eine kombinierte Darstellung der ersten und zweiten Eigenschaften für alle geprüften Fasem zu erhalten, wobei eine der genannten ersten und zweiten Eigenschaften aus der mindestens zweiten Prüfung der gleichen Eigenschaft aus der ersten Prüfung überlagert wird und diese kombinierte Darstellung verschiedene Angaben über Fasem bildet.

2. Verfahren nach Anspruch 1, dadurch gekennzeichnet, dass als erste und zweite Eigenschaften, die Zugkraft und die Dehnungslänge vorgesehen sind.

3. Verfahren nach Anspruch 2, dadurch gekennzeichnet, dass als erste und zweite Darstellung Werte vorgesehen sind, die die Zugkräfte und die Dehnungslängen angeben.

4. Verfahren nach Anspruch 1 und 2, dadurch gekennzeichnet, dass die Kombination der Darstellungen dadurch geschieht, dass für jede Dehnungslänge, aus einer Mehrzahl Dehnungslängen, Zugkräfte addiert werden, die aus der ersten und mindestens zweiten Prüfung stammen.

5. Verfahren nach Anspruch 1, dadurch gekennzeichnet, dass für die Kombination, die ersten und zweiten Darstellungen gemäss der ersten Eigenschaft so ausgerichtet werden, dass für gleiche Eigenschaften in der ersten und zweiten Darstellung die zweite Eigenschaft ausgerichtet ist und dass die ausgerichteten zweiten Eigenschaften der ersten und zweiten Darstellung zusammengerechnet werden um die zweiten Eigenschaften in der kombinierten Darstellung zu bilden.

6. Verfahren nach Anspruch 1, dadurch gekennzeichnet, dass die erste Eigenschaft die Zugkraft auf die Faser und die zweite Eigenschaft die Dehnung oder eine Eigenschaft des Querschnittes der Faser ist.

7. Verfahren nach Anspruch 1, dadurch gekennzeichnet, dass eine dritte Eigenschaft der Fasem bestimmt wird und dass die erste, zweite und dritte Eigenschaft für die gemeinsame Darstellung kombiniert werden.

8. Verfahren nach Anspruch 1, dadurch gekennzeichnet, dass vor der Kombination die erste und die zweite Eigenschaft gespeichert wird.

9. Verfahren nach Anspruch 1, dadurch gekennzeichnet, dass die gemeinsame Darstellung von Werten, die von einzelnen Fasem stammen, benützt wird, um Eigenschaften eines Faserbündels mit mehreren Fasem vorauszusagen.

## Revendications

1. Procédé pour fournir des données de fibres multiples se rapportant à des fibres prises dans un échantillon constitué de fibres, où
un premier test est exécuté pour déterminer des première et deuxième caractéristiques (x,y) d'au moins une fibre prise dans l'échantillon (22) et une première représentation des première et deuxième caractéristiques est réalisée à partir dudit premier test,
au moins un deuxième test est exécuté pour déterminer des première et deuxième caractéristiques (x,y) d'au moins une fibre additionnelle (22) prise dans l'échantillon, et une deuxième représentation des première et deuxième caractéristiques est réalisée à partir dudit deuxième test, caractérisé en ce que
les première et deuxième représentations des première et deuxième caractéristiques sont combinées pour produire une représentation composée des première et deuxième caractéristiques pour toutes les fibres qui ont été testées, où une desdites première et deuxième caractéristiques d'au moins dudit deuxième test est superposée aux mêmes caractéristiques précitées dudit premier test, ladite représentation composée constituant les données de fibres multiples.

2. Procédé selon la revendication 1, caractérisé en ce que lesdites première et deuxième caractéristiques sont constituées par la force de traction et la longueur d'allongement.

3. Procédé selon la revendication 2, caractérisé en ce que lesdites première et deuxième représentations sont constituées par des valeurs représentant lesdites forces de traction et ladite longueur d'allongement.

4. Procédé selon la revendication 1 et 2, caractérisé en ce que la combinaison des caractéristiques est exécutée en additionnant les forces de traction obtenues par ledit premier et au moins deuxième test précités pour chacune d'une pluralité de longueurs de traction.

5. Procédé selon la revendication 1, caractérisé en ce que ladite étape de combinaison comprend:
l'alignement des première et deuxième représentations en accord avec la première caractéristique de telle sorte que, pour des caractéristiques identiques dans les première et deuxième représentations, les deuxièmes caractéristiques seront alignées, et l'addition des deuxièmes caractéristiques alignées des première et deuxième représentations pour former les deuxièmes caractéristiques dans la représentation composée.

6. Procédé selon la revendication 1, caractérisé en ce que ladite première caractéristique est la traction exercée sur la fibre, et ladite deuxième caractéristique est l'allongement ou une caractéristique se rapportant à la section transversale de la fibre.

7. Procédé selon la revendication 1, caractérisé en ce qu'une troisième caractéristique des fibres est déterminée et en ce que lesdites première, deuxième et troisième caractéristiques sont combinées pour produire la représentation composée.

8. Procédé selon la revendication 1, caractérisé en ce que lesdites première et deuxième caractéristiques sont stockées avant la combinaison.

9. Procédé selon la revendication 1, caractérisé en ce que ladite représentation composite de données obtenues à partir de fibres individuelles est utilisée pour prédire les caractéristiques d'un paquet de fibres contenant une pluralité de fibres.
